# EUROPÄISCHE PATENTSCHRIFT

(11) **EP 1 468 701 B1**
(45) Veröffentlichungstag und Bekanntmachung des Hinweises auf die Patenterteilung: **08.06.2005**
(21) Anmeldenummer: 03007122.9
(22) Anmeldetag: 28.03.2003
(51) Int. Cl.: A61L 2/28, C12Q 1/22, G01N 31/22

(54) **Prüfkörper, insbesondere zur Überprüfung der Penetrationseigenschaften eines Sterilisiermittels in Sterilisationsprozessen**
Challenge device, especially for testing the penetration properties of a sterilant in sterilising processes
Dispositif de test, en particulier pour évaluer les proprietés de pénétration d'un stérilisant dans des procédés de stérilisation

(43) Veröffentlichungstag der Anmeldung: 20.10.2004
(73) Patentinhaber: Kaiser, Ulrich, Dr., D-61479 Glashütten (DE)
(72) Erfinder: Kaiser, Ulrich, Dr., D-61479 Glashütten (DE)
(74) Vertreter: Walkenhorst, Andreas

(56) Entgegenhaltungen:
- EP-A- 0 628 814
- EP-A- 0 882 456
- EP-A- 0 982 039
- EP-A- 1 084 714
- WO-A-97/12637
- DE-U- 8 700 471
- DE-U- 29 811 381
- US-A- 5 872 004

## Beschreibung

Die Erfindung bezieht sich auf einen Prüfkörper, insbesondere zur Überprüfung der Penetrationseigenschaften eines Sterilisiermittels, bei dem ein zur Aufnahme eines Indikators vorgesehenes Detektorvolumen über einen Gassammelraum mit einer Sterilisationskammer verbindbar ist. Sie betrifft weiterhin die Verwendung eines derartigen Prüfkörpers. Derartige Prüfkörper sind beispielsweise aus der EP 0 628 814 A1 oder aus der EP 1 172 117 A2 bekannt.

Bei aseptischen Anwendungen wie bei Operationen im Krankenhaus ist die Verwendung von sterilen Werkzeugen, Instrumenten oder Materialien zwingend erforderlich. Bei einer dazu notwendigen Sterilisation wird üblicherweise das sterilisierende Medium, wie beispielsweise Dampf, Formaldehyd, Ethylenoxid, Wasserstoffperoxid und/oder Ozon, über die Gasphase auf die Oberfläche des zu sterilisierenden Instruments übertragen, um die totale Abtötung vorhandener Keime zu gewährleisten. Zu diesem Zweck kommen normalerweise Sterilisatoren mit Sterilisationskammern zum Einsatz, in die die zu sterilisierenden Instrumente oder Materialien - im Regelfall verpackt - eingelegt werden. Zur eigentlichen Sterilisation wird die Sterilisationskammer mit gasförmigem Sterilisiermittel - auch als sterilisierendes Agens bezeichnet - geflutet, wobei zuvor die Entfernung der Luftatmosphäre gewährleistet sein muß. Das Sterilisiermittel soll die Oberflächen der zu sterilisierenden Instrumente oder Materialien kontaktieren, so dass die erwünschte Keimabtötung eintritt.

Da die vollständige Sterilisation der Güter an allen Stellen nur dann sichergestellt ist, wenn das Sterilisiermittel auch alle inneren Oberflächen erreicht, z. B. poröse Güter oder Hohlkörper ist zu Beginn des Sterilisationsprozesses die Entfernung der im Innern der Güter und im Innern des Sterilisierraums befindlichen Luft durch ein geeignetes Entlüftungsverfahren zu gewährleisten. Anschließend wird die Sterilisationskammer mit dem Sterilisiermittel geflutet, um im Innern der Sterilisationskammer alle Oberflächen der Gerätschaften mit dem Sterilisiermittel zu erreichen. Dies ist nur möglich, wenn die vollständige Penetration des Sterilisiermittels an alle Oberflächen gewährleistet ist.

Als problematisch bei derartigen Sterilisationsmaßnahmen hat sich jedoch die Komplexität beim strukturellen Aufbau medizinischer Instrumente erwiesen. Gerade bei medizinischen Instrumenten kommen nämlich zunehmend Gerätschaften zum Einsatz, bei denen Röhren oder Schläuche mit relativ großer Länge und vergleichsweise geringem freien Querschnitt verwendet werden, so dass ein zuverlässiges Benetzen des Sterilisiermittels zu sämtlichen inneren Oberflächen erschwert ist, wenn sich dort andere Gase befinden. Des Weiteren können Materialien oder Güter mit komplexer Innenoberfläche, wie beispielsweise Textilpakete, zu sterilisieren sein. In derartigen Fällen können eventuell vorhandene Ansammlungen von Restluft oder anderen nicht kondensierbaren Gasen (NKG) den durchgängigen Kontakt der behandlungsbedürftigen Oberfläche ganz oder teilweise verhindern. Die vollständige Sterilisation ist hingegen nur dann sichergestellt, wenn die sich im innem der Güter befindliche Luft zu Beginn der Sterilisationsmaßnahme vollständig entfernt wird, keine Luft durch Undichtigkeiten während der Vakuumphase eindringt und/oder keine nicht kondensierbare Gase in die Sterilisationskammer mit dem Sterilisiermittel eingetragen werden, damit das Sterilisiermittel an alle behandlungsbedürftigen Oberflächen gelangt.

Da zudem die Sterilität von Instrumenten vor ihrem Gebrauch nicht direkt geprüft werden kann, sind bei der Inbetriebnahme von Sterilisationsprozessen die Validierung und während des Betriebs des Sterilisationsprozesses Routinekontrollen notwendig. Dazu kommen Detektoren zum Einsatz, die den Sterilisationserfolg nachweisen müssen. Beispielsweise können Chemo-Indikatoren zum Einsatz kommen, die im Falle einer Benetzung ihrer Oberfläche durch das Sterilisiermittel, wie beispielsweise Dampf, ihre Farbe ändern, so dass direkt erkennbar ist, dass an der Stelle des Chemo-Indikators ein Kontakt tatsächlich stattgefunden hat. Alternativ oder zusätzlich können auch Bio-Indikatoren eingesetzt werden, die in Form von Streifen, Suspensionen oder selbst entwickelnden Keimkulturen oder Gemische verschiedener Keimkulturen zum Einsatz kommen, bei denen nach der Durchführung der Sterilisationsmaßnahme überprüft wird, ob alle Keime vollständig inaktiviert wurden.

Bei der Verwendung derartiger Indikatoren wird überprüft, ob am tatsächlichen Einsatzort des Indikators innerhalb der Sterilisationskammer eine aktive Benetzung der Indikatoroberfläche mit Sterilisiermittel eingetreten ist. Bei der Verwendung dieser Indikatoren ist jedoch ein direkter Nachweis des Sterilisationserfolgs auch an vergleichsweise unzugänglichen Innenoberflächen komplexer Instrumente nicht möglich, da diese an den kritischen Stellen nicht plaziert werden können. Daher werden gemeinsam mit den behandlungsbedürftigen Gütern Prüfkörpersysteme mit sterilisiert, durch die der Sterilisationserfolg ermittelt wird. Beispielsweise wurde für die Sterilisationsmaßnahme bei Textilien oder anderen Materialien von Bowie und Dick (Bowie, I. W., e. a., The Bowie + Dick autoclave tape test, Lancet I, 1963, S. 585-587) ein Standardtest angegeben, bei dem in einem Wäschepaket von 6,6 kg Gewicht zentral ein Chemo-Indikatorblatt von DINA 4-Größe plaziert wird. Allerdings ist dieser Standardtest aufgrund der Wäschequalität, Wäschehistorie und Packungsindividualität nicht reproduzierbar und hat andere Penetrationseigenschaften als Hohlkörper.

Alternativ können so genannte Prüfkörper oder Prüfkörpersysteme eingesetzt werden. Bei einem derartigen Prüfkörpersystem, wie es beispielsweise in der EP 0 628 814 A1 oder in der EN 867-5 beschrieben ist, wird die unzugängliche Innenoberfläche komplexer Instrumente durch ein geeignetes Modell nachgebildet, über das der Erfolg der Penetrationsvorgänge in analoger Weise auch für komplexe Instrumente überprüft werden kann.

Bei diesen bekannten, auch als "Process Challenge Device" (PCD) bezeichneten Prüfkörpersystemen ist ein zum Nachweis eines erfolgten Kontakts durch das Sterilisiermittel geeignet ausgebildeter Detektor gaseingangsseitig mit einem in seiner Länge geeignet gewählten, an seinem Einlassende offen gehaltenen Schlauch verbunden. Das Hohlkörpersystem simuliert dabei das Verhalten ähnlich gestalteter Instrumente, die zur Sterilisation vorgesehen sind, wobei insbesondere bei alternierendem Medienaustausch nach Art eines fraktionierten Vakuums und/oder bei der Kondensation von Dampf eventuell noch vorhandene Restluft oder andere nicht kondensierbare Gase am Schlauchende und somit im Bereich des Detektors aufkonzentriert werden. Der Schlauch wirkt somit als Gassammelraum für Restluft oder andere nicht kondensierbare Gase, wobei der Detektor über diesen Gassammelraum mit der Sterilisationskammer verbunden ist.

Falls bei der Verwendung eines derartigen Systems ein Kontakt des am Schlauchende angeordneten Detektors mit Sterilisiermittel festgestellt wird, kann davon ausgegangen werden, dass - gegebenenfalls unter Berücksichtigung eines geeignet gewählten Sicherheitsaufschlags in Bezug auf die Penetrationseigenschaften - in den zu sterilisierenden Instrumenten auch an deren unzugänglichsten Stellen ihrer Innenoberfläche ein Kontakt mit Sterilisiermittel erfolgt sein muss. Ein derartiges Schlauchmodell als Prüfkörper, das als Detektor beispielsweise Bio- oder Chemo-Indikatoren aufnehmen kann, ist auch in der Euronorm EN 867-5 bei der Überprüfung von Sterilisationsprozessen vorgesehen. Bei noch komplexeren zu sterilisierenden Gütern können in ihrer Dimensionierung geeignet angepasste Prüfkörper anderer Bauart eingesetzt werden, wie dies beispielsweise in den Euronormen EN 285, EN 14180, EN 1422 oder EN 867-5 beschrieben ist.

Der Einsatz derartiger Prüfkörper ermöglicht auch, unter bestimmten Randbedingungen physikalische Messmethoden einzusetzen. Beispielsweise ist aus der EP 1 172 117 A2 ein Prüfsystem für Sterilisationsmaßnahmen mit einem Prüfkörper bekannt, bei dem als Nachweis für eine erfolgte Benetzung einer Innenoberfläche durch Sterilisiermittel die mit der lokalen Kondensation von Dampf an der zu überwachenden Stelle verbundene lokale Temperaturänderung gemessen wird. Der in diesem System eingesetzte Prüfkörper ist demzufolge hinsichtlich seiner Wärmeleiteigenschaften besonders geeignet ausgestaltet.

Allerdings ist die erreichbare Messgenauigkeit dieser Systeme nur begrenzt. Insbesondere bei der Modellierung vergleichsweise komplexer oder für das Sterilisiermittel schwer zugänglicher Instrumente ist die erforderliche Messempfindlichkeit - falls überhaupt - nur erreichbar, indem ein vergleichsweise voluminös ausgebildeter Gassammelraum verwendet wird. Bei der Verwendung von Schlauchmaterial zur Bildung des Gassammelraums können Wärmeübergänge über die Schlauchwand auftreten und das Ergebnis verfälschen, sofern thermoelektrische Messungen als Detektoren eingesetzt werden.

Der Erfindung liegt daher die Aufgabe zugrunde, einen Prüfkörper der oben genannten Art anzugeben, mit dem bei kompakter Bauweise eine besonders hohe Nachweisempfindlichkeit erreichbar ist.

Diese Aufgabe wird dadurch gelöst, dass der Gassammelraum mehrstufig ausgeführt ist, wobei der Querschnitt und das Volumen jeder Stufe zum Detektor hin abnimmt.

Die Erfindung geht dabei von der Überlegung aus, dass bei üblichen Prüfkörpern eine hohe Nachweisempfindlichkeit nur mit entsprechend lang dimensioniertem Gassammelraum und somit gerade auf Kosten der Kompaktheit erreichbar ist. Weiterhin nachteilig ist, dass bei langen dünnen Schläuchen die Kondensation von Dampf in Dampf-Sterilisationsprozessen stattfinden kann. Für eine kompakte Bauweise sollte daher unter Verwendung von ansonsten üblichen Indikatoren, wie beispielsweise physikalischen Methoden, Bio- oder Chemo-Indikatoren, die Gaszuführung zum Detektorvolumen derart ausgestaltet sein, dass die Absonderung von Restluft oder anderen nicht kondensierbaren Gasen im Sterilisiermittel durch die Verwendung größerer Volumina erreicht wird und dann über die Querschnittsverkleinerung dem Detektor zugeführt wird. Eine derartige Verstärkung ist erreichbar durch eine gezielte Aufkonzentration der Luft- oder Gasreste im Zuströmbereich zum Detektorvolumen. Dazu ist der Gassammelraum des Prüfkörpers zumindest zweistufig, gegebenenfalls aber auch mehrstufig, ausgestaltet, wobei sich die Stufen hinsichtlich ihrer Dimensierung, also insbesondere hinsichtlich ihres jeweiligen Volumens und/oder Querschnitts, voneinander unterscheiden. Die dem Detektorvolumen unmittelbar benachbarte Stufe kann dabei dazu dienen, die Zugänglichkeit zu den jeweiligen besonders unzugänglichen Innenoberflächen des behandlungsbedürftigen Instruments zu simulieren. Die Aufkonzentration der Restluft in dieser Stufe und somit unmittelbar benachbart zum Detektorvolumen wird dabei für eine hohe Empfindlichkeit durch die in Richtung zur Sterilisationskammer gesehen weiteren Stufen des Gassammelraums begünstigt, die als Kondensationszone zur selektiven Auskondensation von Sterilisiermittel dienen, sofern Dampf - Sterilisationsprozesse zum Einsatz kommen.

Für eine besonders hohe Wirksamkeit der selektiven Aufkonzentration noch vorhandener Restluft in den dafür vorgesehenen Stufen des Gassammelraums und somit eine besonders hohe Nachweisempfindlichkeit nimmt der Querschnitt zwischen benachbarten Stufen vorteilhafterweise in Richtung zum Detektorvolumen hin mindestens um 50%, vorzugsweise um mehr als 75%, ab. Somit ergibt sich ein Gasströmungspfad in den Prüfkörper, bei dem ein erster Strömungsraum mit vergleichsweise großem Volumen und Querschnitt mit dem Innenraum der Sterilisierkammer verbunden ist, wobei sich Volumen und Querschnitt im zweiten Strömungsraum zum Detektor hin verringern. In weiterer vorteilhafter Ausgestaltung ist das Volumen der Stufe zum Detektor hin kleiner gehalten als das Volumen der Stufe zur Sterilisationskammer hin. Der dem Detektor innerhalb des Prüfkörpers vorgeschaltete Strömungskanal für das Gas entspricht somit der gasstromseitigen Hintereinanderschaltung eines weiten und eines engen Kanalabschnitts.

Für eine besonders hohe Nachweisgenauigkeit ist der Detektor im Gassammelraum vorteilhafterweise derart gestaltet, dass die Anwesenheit auch nur geringfügiger Restluftmengen eine Beaufschlagung mit Sterilisiermittel verhindern würde. Da insbesondere bei einer Betriebsweise mit alternierenden Medien sich infolge des Gasaustauschs noch verbliebene Restluftmengen im Endbereich des Sondenkanals ansammeln, ist der Detektor notwendigerweise in einem Bereich an dem der Mündung gegenüberliegenden Ende des Gassammelraums angeordnet.

Der Prüfkörper kann hinsichtlich der Dimensionierung seiner wesentlichen Bestandteile an vorgegebene normierte Prüfverfahren oder an reale behandlungsbedürftigen Güter angepasst werden. Zu diesem Zweck beträgt der Querschnitt der dem Detektorvolumen unmittelbar benachbarten Stufe des Gassammelraums vorteilhafterweise etwa 1 bis 20 mm², wobei der Gassammelraum in dieser Stufe in weiterer oder alternativer vorteilhafter Ausgestaltung eine Kanallänge von zumindest 10 cm, vorzugsweise von etwa 30 bis 100 cm, aufweist. Die Stufen des Gassammelraums können dabei eine geeignete Querschnittsform, insbesondere einen runden oder eckigen Querschnitt, aufweisen und jeweils aus einem Metalloder Kunststoffrohr oder -behälter gebildet sein, so dass sich im Vergleich zu Schläuchen eine hohe Festigkeit und gute Haltbarkeit ergibt.

Eine besonders kompakte Bauweise ist erreichbar, indem eine erste Stufe des Gassammelraums in der Art einer ineinander geschachtelten Bauweise im Wesentlichen innerhalb einer zweiten Stufe des Gassammelraums angeordnet ist. Die zweite Stufe des Gassammelraums ist dabei in weiterer vorteilhafter Ausgestaltung als die erste Stufe umschließendes Außengehäuse ausgebildet, so dass die dem Detektorvolumen unmittelbar benachbarte Stufe des Gassammelraums innerhalb des Außengehäuses geführt wird. Das Außengehäuse kann dabei Als beliebiger Hohlraum ausgeführt sein, der zur gasseitigen Verbindung mit der Sterilisationskammer mit geeignet plazierten Durchtrittsöffnungen versehen ist.

In alternativer vorteilhafter Ausgestaltung ist eine kompakte Bauweise aber auch dadurch erreichbar, dass eine erste Stufe des Gassammelraums vorteilhafterweise um oder in eine weitere Stufe des Gassammelraums bildendes Außengehäuse herumgeführt ist, wobei sich die erste Stufe insbesondere spiralartig um oder in das Außengehäuse herumwinden kann.

Im Übrigen kann der Gassammelraum hinsichtlich der Strömungscharakteristik in seinem Innenbereich an weitere Vorgaben angepasst sein. Dazu ist der Gassammelraum zweckmäßigerweise in zumindest einer Stufe mit porösem Material gefüllt, wobei als poröses Material insbesondere Zellulose-, Baum-, Glas-, Steinoder Metallwolle gewählt sein kann. Das poröse Material ist dabei vorteilhafterweise insbesondere innerhalb einer als Außengehäuse ausgeführten Stufe des Gassammelraums angeordnet, um die Konvektion im Gassammelraum möglichst niedrig zu halten und Rückvermischungen zu vermeiden.

Vorteilhafterweise ist auch der Detektor des Prüfkörpers an sich bereits für eine besonders hohe Nachweisempfindlichkeit ausgelegt. Dazu umfasst der Detektor vorzugsweise einen im gasseitig mit dem Sondenkanal verbundenen Detektorvolumen angeordneten Indikator. Das Detektorvolumen ist dabei vorzugsweise besonders gering gehalten und weitgehend an das vom eigentlichen Indikator eingenommene Volumen angepasst. Vorteilhafterweise ist das Detektorvolumen dabei kleiner als etwa 250 - 500 µl gewählt, so dass bei der Verwendung eines üblichen Chemo- oder Bioindikators mit Papier als Träger, der ein Volumen von 100 - 250 µl einnimmt, nahezu die Hälfte des Detektorvolumens durch den eigentlichen Indikator ausgefüllt ist. Dabei sind vorzugsweise die Stufen des Gassammelraums sowie der Detektor aus Metall oder Kunststoff oder einer Metall-Kunststoff-Verbindung mit jeweils angepaßten, unterschiedlichen Wandstärken aufgebaut.

Als Detektor kann ein System zur Ermittlung physikalischer Kennwerte, wie beispielsweise ein Feuchtigkeits-, Temperatur-, Druck- und/oder Ultraschallsensor, der sich im Sterilisatorraum befindet, zum Einsatz kommen, der bedarfsweise auch als sogenannter Datenlogger für eine drahtlose Übermittlung der aufgenommenen Daten ausgestaltet sein kann. Auch können beispielsweise Festkörper wie zum Beispiel Salze eingesetzt werden, die sich bei Anwesenheit des Sterilisiermittels physikalisch verändern, also beispielsweise ihren Schmelzpunkt oder ihre Farbe ändern. Vorteilhafterweise ist als Indikator aber ein Chemo-Indikator, der bei Kontakt mit dem eingesetzten Sterilisiermittel seine Farbe ändert, oder ein Bio-Indikator, beispielsweise in Form von Indikatorstreifen und/oder selbstentwickelnden Bio-Indikatoren, vorgesehen.

Der Prüfkörper ist besonders zur Überwachung von Sterilisationsprozessen mit gasförmigem Sterilisiermittel wie beispielsweise Niedertemperatur-Dampf-, Formaldehyd-, Ethylenoxid-, Wasserstoffperoxid- oder Ozon-Sterilisationsprozessen geeignet. Aufgrund der durch die gasseitige Hintereinanderschaltung mehrerer Stufen des Gassammelraums vor dem Detektor erreichten spezifischen Strömungseigenschaften und der Bereitstellung einer Kondensationszone zur spezifischen Auskondensation des Sterilisiermittels eignet sich der Prüfkörper aber auch in besonderem Maße zum Einsatz in einem Sterilisationsverfahren, bei dem Dampf als Sterilisiermittel eingesetzt wird. Vorteilhafterweise wird der Prüfkörper daher zur Überwachung von Dampf-Sterilisationsprozessen verwendet. Grundsätzlich können dabei alle Entlüftungsvarianten für die Sterilisationskammer eingesetzt werden. Dabei klärt der Prüfkörper im Prozess den Betreiber auf, welche Dampfdurchdringungseigenschaften der Prozess hat.

Die mit der Erfindung erzielten Vorteile bestehen insbesondere darin, dass durch die gasseitige Hintereinanderschaltung mehrerer Stufen des Gassammelraums mit unterschiedlicher Dimensionierung insbesondere bei der Verwendung von Dampf als Sterilisiermittel eine Kondensationszone zur selektiven lokalen Auskondensation von Sterilisiermittel bereitgestellt wird, die zu einer lokalen Aufkonzentration von Restluft und/oder nicht kondensierbaren Gasen im Bereich der dem Detektorvolumen benachbarten Stufe des Gassammelraums führt. Damit ist insbesondere auch bei wechselnder Evakuierung und Neubeaufschlagung mit Dampf in der Art eines integrierenden Systems eine Auswertung kumulierter Mengen von Restluft und/oder nicht kondensierbaren Gasen ermöglicht, die eine Verstärkungswirkung bei der Detektion sicherstellt. Die Restluft verbleibt somit auch bei einer Druckreduzierung der Sterilisationskammer im Mündungsbereich des Gassammelraums, ohne dass dies durch in der Sterilisationskammer eventuell auftretende Konvektions- oder andere Strömungen beeinträchtigt würde. Bei einem erneuten Beaufschlagen der Sterilisationskammer mit Dampf wird die im Gassammelraum enthaltene Restluft - soweit vorhanden - erneut in die dem Detektor unmittelbar benachbarte Stufe hineingedrückt und gelangt dort insbesondere bis in den Bereich unmittelbar in der Nähe des Detektors. Somit werden auch besonders geringe Restluftmengen noch gezielt in den Nachweisbereich des Detektors geführt, so dass die Nachweisempfindlichkeit gegenüber der vorhandenen Restluft besonders hoch ist. Eine Benetzung des im Detektor vorgehaltenen Indikators mit Sterilisiermittel erfolgt somit erst dann, wenn auch in sämtlichen anderen vergleichsweise komplexen Hohlkörpern eine zuverlässige Sterilisation stattfindet.

Durch den damit erreichten Verstärkungseffekt kann eine hohe Nachweisempfindlichkeit auch bei kompakter Bauweise sichergestellt werden, wobei zudem der Einsatz besonders robuster und alterungsbeständiger Materialien für den Gassammelraum möglich ist. Der erreichbare Verstärkungseffekt ist dabei derart hoch, dass bei kompakter Bauweise selbst nur sehr schwer penetrierbare Instrumente wie beispielsweise Trokare, Atroskopie - Instrumente oder mehrkanalige Endoskope zuverlässig nachgebildet werden können. Durch die mehrstufige Ausführung sind darüber hinaus mehrere Freiheitsgrade für die Nachbildung realer Instrumente vorhanden, so dass auch poröse oder Hohlkörperkonstruktionen gut simuliert werden können.

Ein Ausführungsbeispiel der Erfindung wird anhand einer Zeichnung näher erläutert. Darin zeigen:
- FIG 1: ein Aufbauschema für einen Prüfkörper, und
- FIG 2 bis 5: jeweils einen Prüfkörper zur Überprüfung der Penetrationseigenschaften eines Sterilisiermittels.

Gleiche Teile sind in allen Figuren mit den selben Bezugszeichen versehen.

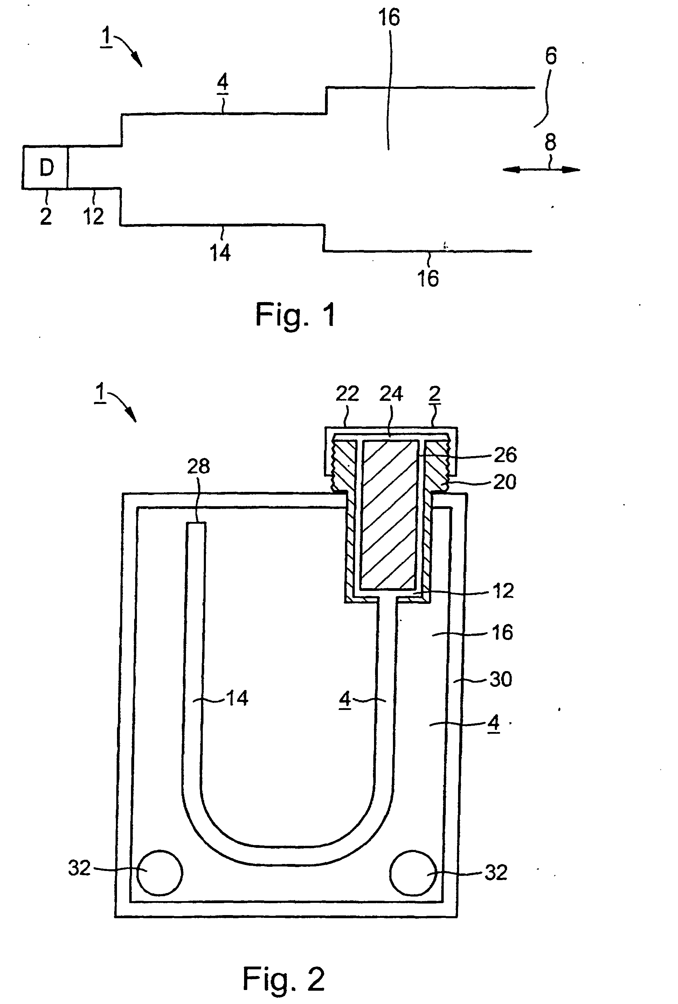

Der Prüfkörper 1 mit dem in Figur 1 dargestellten Aufbauschema ist zur Überprüfung der Penetrationseigenschaften eines Sterilisiermittels ausgelegt. Die beim Einsatz des Prüfkörpers 1 gewonnenen Erkenntnisse können insbesondere zur Verifizierung oder Überprüfung von Sterilisationsmaßnahmen in einem Dampfsterilisationsverfahren herangezogen werden. Bei einer derartigen Dampfsterilisation werden die zu sterilisierenden Instrumente oder Materialien in eine nicht näher dargestellte Sterilisationskammer eingebracht. Zur Sterilisation wird die Sterilisationskammer zunächst entlüftet. Der Entlüftungsprozess kann dadurch Strömungsverfahren, Über- oder Unterdruck-Entlüftungszyklen oder deren Kombination erfolgen.

Um bei einem derartigen, auf fraktioniertem Vakuum beruhenden Entlüftungsverfahren nachweisen zu können, dass bei den zu sterilisierenden Instrumenten und Materialien auch tatsächlich sämtliche inneren Oberflächen mit Sterilisiermittel benetzt worden sind und somit die erforderliche vollständige Penetration stattgefunden hat, wird der Prüfkörper 1 beispielsweise zur Validierung der Sterilisationsmaßnahme bei ihrer Inbetriebnahme oder bei Routinekontrollen während des Sterilisationsprozesses mit in der Sterilisationskammer eingesetzt. Der Prüfkörper 1 umfasst einen Detektor 2, der für den gezielten Nachweis ausgelegt ist, dass eine Benetzung durch Sterilisiermittel tatsächlich stattgefunden hat.

Der Prüfkörper 1 ist dafür ausgebildet, die ordnungsgemäße Benetzung auch vergleichsweise schwer zugänglicher innerer Oberflächen von Instrumenten oder Materialien geeignet nachzubilden. Dazu ist dem Detektor 2 ein Gassammelraum 4 vorgeschaltet, dessen Endmündung 6 offen gehalten ist, so dass der Detektor 2, wie durch den Doppelpfeil 8 angedeutet, über den Gassammelraum 4 gasseitig mit der Sterilisationskammer verbunden ist. Der Prüfkörper 1 ist dabei für eine besonders kompakte Bauweise bei gleichzeitig besonders hoher Nachweisempfindlichkeit hinsichtlich der Penetrationseigenschaften der zu simulierenden Instrumente oder Gerätschaften ausgebildet. Dazu ist der Gassammelraum 4 mehrstufig ausgeführt, wobei im Ausführungsbeispiel eine dem Detektor 2 unmittelbar benachbarte erste Stufe 12 gasseitig mit weiteren Stufen 14, 16 in Reihe geschaltet ist. Selbstverständlich können in Ergänzung einer derartigen dreistufigen Ausführung auch noch weitere Stufen hintereinandergeschaltet sein, oder es kann eine zweistufige Ausführung vorgesehen sein.

Der Prüfkörper 1 ist in besonderem Maße für eine Anwendung bei einem dampfbasierten Sterilisationsverfahren unter Verwendung einer Sterilisationskammer mit besonders hoher Nachweisgenauigkeit ausgelegt. Dazu wird der Prüfkörper 1 gemeinsam mit den zu sterilisierenden Instrumenten oder Materialien in die Sterilisationskammer eingebracht. Im ersten Bearbeitungsschritt, in dem die Sterilisationskammer evakuiert wird, wird auch der mehrstufige ausgebildete Gassammelraum 4 evakuiert. Anschließend, wenn die Sterilisationskammer mit Dampf als Sterilisiermittel befüllt wird, strömt der Dampf über die Endmündung 6 auch in die dritte Stufe 16 und von dieser aus in die zweite und erste Stufe 12, 14 des Gassammelraums 4 ein. Eventuell noch vorhandene Restluft, die durch die Bildung von Luftpolstern eine zuverlässige Sterilisation der Instrumente oder Materialien verhindern könnte, wird dabei über die dritte Stufe 16 in die zweite und erste Stufe 12, 14 des Gassammelraums 4 hineingedrückt und sammelt sich an dessen der Endmündung 6 gegenüber liegendem Ende. Somit erfolgt eine Aufkonzentration der Restluft im Bereich des Detektors 2, so dass eine vollständige Benetzung eines im Detektor angeordneten Indikators mit dem als Sterilisiermittel vorgesehen Dampf unterbleibt.

In sukzessiv erfolgenden weiteren Verfahrensschritten, bei denen in der Art eines fraktionierenden Vakuums erneut ein Unterdruck in der Sterilisationskammer erzeugt und diese anschließend mit Dampf als Sterilisiermittel befüllt wird, erfolgt ein sukzessiv zunehmender Austrag der Restluft aus der Sterilisationskammer. Dadurch erfolgt eine zunehmend zuverlässige und durchgängige Benetzung sämtlicher inneren Oberflächen der Instrumente, die analog auch zu einer zunehmend besseren Benetzung der Innenoberfläche der ersten Stufe 12 des Gassammelraums 4 führt. Falls eine ausreichende Penetration des Sterilisiermittels in den Prüfkörper 1 hinein erfolgt, kann eine durchgängige Benetzung auch des Indikators festgestellt werden. Wenn dies eintritt, wird die Sterilisationsmaßnahme als erfolgreich angesehen.

Durch die mehrstufige Ausgestaltung des Gassammelraums 4 mittels der gasseitigen Vorschaltung der zweiten und dritten Stufe 14, 16 vor die erste Stufe 12 und durch die geeignet gewählte Dimensionierung der Stufen 12, 14, 16 ist beim Prüfkörper 1 sichergestellt, dass die für den Prozess schädliche Restluft im unmittelbaren Bereich der ersten Stufe 12 gezielt aufkonzentriert wird. Diese Aufkonzentration erfolgt insbesondere durch die gasseitig vorgelagerten Stufe 14, 16, die als Kondensationszone für das Sterilisiermittel dient und eine selektive lokale Auskondensation des Sterilisiermittels unmittelbar vor dem Eintrittsbereich in die erste Stufe 12 bewirkt. Durch die damit erreichte Aufkonzentration der Restluft oder anderer nicht kondensierbarer Gase erfolgt ein nachhaltiger und besonders zuverlässiger Nachweis der Restluft oder andere nicht kondensierbare Gase, so dass der Prüfkörper 1 mit einer besonders hohen Nachweisgenauigkeit betrieben werden kann.

Ein nach dem Aufbauschema gemäß Figur 1 aufgebauter Prüfkörper 1 ist in Figur 2 geschachtelt gezeigt. Dessen Detektor 2 umfasst eine mit einem Gewinde versehene Detektorhülse 20, die mit einem aufgeschraubten Deckel 22 verschließbar ist. Innerhalb der Detektorhülse 20 ist ein Detektorvolumen 24 von im Ausführungsbeispiel etwa 240 µl vorgesehen, in das ein auswechselbarer Indikator 26 eingebracht ist.

Der Indikator 26, der im Ausführungsbeispiel ein Volumen von etwa 120µl einnimmt und das Detektorvolumen 24 somit annähernd zur Hälfte ausfüllt, könnte als Sensor zur Ermittlung eines physikalischen Messwerts, beispielsweise der Temperatur oder des Drucks, ausgelegt sein. Im Ausführungsbeispiel ist als Indikator 26 jedoch ein Bio- oder Chemo-Indikator vorgesehen. Bei der Ausgestaltung als Bio-Indikator ist der Indikator 26 dabei an seiner Oberfläche mit keimfähigen Kulturen versetzt, die bei einer ordnungsgemäßen Benetzung mit Dampf als Sterilisiermittel abgetötet werden. Bei dieser Ausgestaltung wird der Indikator 26 also derart eingesetzt, dass nach erfolgter Sterilisation eine Überprüfung auf noch vermehrungsfähige Keime vorgenommen wird. Falls solche festgestellt werden, wird auf unzureichende Sterilisation geschlossen.

Bei einer Ausgestaltung des Indikators 26 als Chemo-Indikator ist dieser derart ausgebildet, dass er bei einer Benetzung seiner Oberfläche mit Dampf als Sterilisiermittel seine Farbe ändert, so dass durch optische Kontrolle direkt auf eine durchgängige Benetzung geschlossen werden kann. Bei Nicht-Benetzung erfolgt kein oder ein anderer Farbumschlag. Die erste Stufe 14 des Gassammelraums 4 ist im Wesentlichen aus einer lang gestreckten Röhre mit rundem oder auch eckigem Querschnitt und bevorzugt aus Metall oder Kunststoff gebildet. Im Ausführungsbeispiel nach Figur 2 weist die dem Detektor 2 unmittelbar benachbarte erste Stufe 14 als lichte Weite einen Innendurchmesser von 2 mm auf, so dass ihr Strömungsquerschnitt etwa 3,2 mm² beträgt. Als Kanallänge ist etwa 50 cm gewählt. Somit weist die erste Stufe 14 ein Innenvolumen von etwa 1,75 ml auf.

Der Detektor 2 ist dabei am einer Mündung 28 für das Gas gegenüberliegenden Ende der ersten Stufe 14 angeordnet. Die erste Stufe 14 ist im Ausführungsbeispiel u-förmig und somit geschwungen ausgeführt und in ihrer Gesamtheit innerhalb eines den Innenraum der zweiten Stufe 16 umschließenden Außengehäuses 30 angeordnet, so dass sich eine geschachtelte Bauweise für den Prüfkörper 1 ergibt. Das Außengehäuse 30 kann dabei beispielsweise im Wesentlichen als Metall- oder Kunststoffrohr mit etwa 20 cm Länge und 25 mm Innendurchmesser ausgestaltet sein, so dass sich ein Volumen der zweiten Stufe 16 von etwa 0,1 l ergibt. Somit nehmen Strömungsquerschnitt und Volumen der Stufen 14, 16 zwischen den benachbarten Stufen 14, 16 in Strömungsrichtung zum Detektor 2 hin signifikant ab.

Die zweite Stufe 16 kommuniziert gasseitig über eine Anzahl von im Außengehäuse 30 angeordneten Eintrittsöffnungen 32 mit der Umgebungsatmosphäre. Gerade bei einer rohrförmigen Ausgestaltung des Außengehäuses 30 kann aber auch dessen kompakter Bodenbereich zur Bildung einer vergleichsweise großdimensionierten Eintrittsöffnung 32 offengehalten sein. Die Mündung 28, über die die erste Stufe 14 gasseitig mit dem die zweite Stufe 16 des Gassammelraums 4 bildenden Innenvolumen des Außengehäuses 30 verbunden ist, ist dabei innerhalb der Außengehäuses 30 an einer Position angeordnet, an der im Hinblick auf die Positionierung der Eintrittsöffnungen 32 auch bei Gaswechseln mit nur geringen Gasströmungsintensitäten zu rechnen ist, so dass die gewünschte lokale Aufkonzentration von Gasresten durch lokale selektive Auskondensation des Sterilisiermittels noch weiter begünstigt ist. Im Ausführungsbeispiel nach Figur 2, bei dem die Eintrittsöffnungen 32 in den unteren Ecken des Außengehäuses 30 vorgesehen sind, erweist sich anhand dieser Kriterien eine Positionierung der Mündung 28 im oberen Bereich des Innenvolumens als günstig.

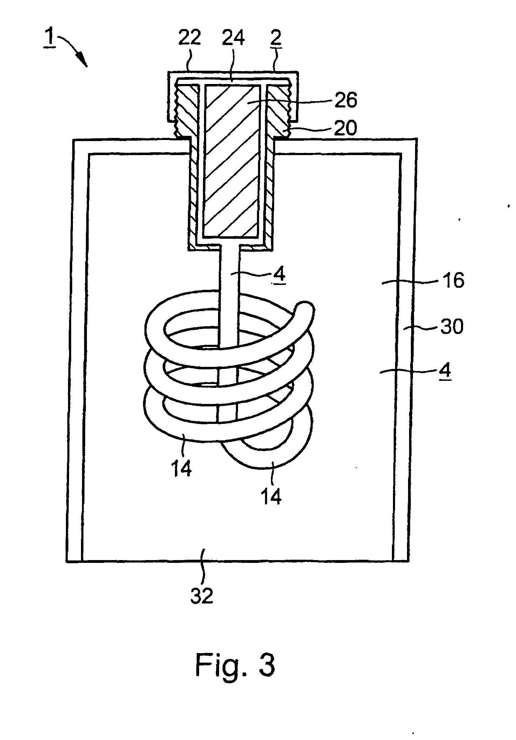

Alternative Ausführungsformen für den Prüfkörper 1 sind schematisch in den Figuren 3 bis 5 dargestellt. Beim Prüfkörper 1 nach Figur 3 ist die erste Stufe 14 des Gassammelraums 4 ebenfalls in der Art einer geschachtelten Bauweise innerhalb der zweiten Stufe 16 des Gassammelraums 4 geführt. Der Prüfkörper 1 ist in diesem Fall jedoch für eine besonders hohe Nachweisempfindlichkeit bei besonders kompakter Bauweise ausgeführt. Um die für eine hohe Nachweisempfindlichkeit günstige vergleichsweise große Kanallänge der ersten Stufe 14 auch bei kompakter Bauweise herzustellen, ist die erste Stufe 14 in dieser Ausführungsform in der Art einer Wendel innerhalb des die zweite Stufe 16 umschließenden Außengehäuses 30 geführt. Als Eintrittsöffnung 32 ist in diesem Fall der komplette Bodenbereich des Außengehäuses 30 offen gehalten. Im Hinblick auf die genannten Kriterien muss die Positionierung der Mündung 28 nahe dem Deckelbereich des Außengehäuses 30 erfolgen.

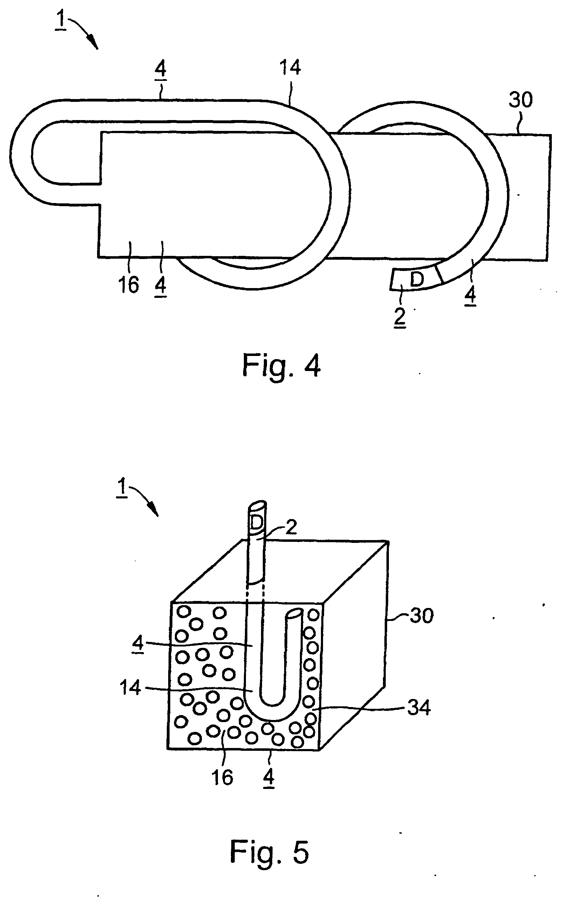

Beim Prüfkörper 1 nach Figur 4 ist die erste Stufe 14 des Gassammelraums 4 nicht innerhalb, sondern vielmehr außerhalb der zweiten Stufe 16 angeordnet und spiralartig um das Außengehäuse 30 der zweiten Stufe 16 herumgeführt. Bei geeignet dicker Wandstärke des Außengehäuses 30 kann die erste Stufe 14 in dieser Ausführungsform auch vollständig in die Außenwand integriert sein.

In der Ausführungsform nach Figur 5 weist der Prüfkörper 1 ein im Wesentlichen kastenförmig ausgebildetes Außengehäuse 30 zur Begrenzung des Gassammelraums 4 auf. Der im Wesentlichen u-förmig ausgestaltete erste Stufe 14 ist in diesem Fall ebenfalls nahezu vollständig im Innenraum der zweiten Stufe 16 geführt. Beim Prüfkörper 1 ist die zweite Stufe 16 zudem mit porösem Material 34, insbesondere mit Zellulose-, Baum-, Glas-, Stein- oder Metallwolle, gefüllt.

### Bezugszeichenliste

- 1: Prüfkörper
- 2: Detektor
- 4: Gassammelraum
- 6: Endmündung
- 8: Doppelpfeil
- 12, 14, 16: Stufe
- 20: Detektorhülse
- 21: Deckel
- 24: Detektorvolumen
- 26: Indikator
- 28: Mündung
- 30: Außengehäuse
- 32: Eintrittsöffnung
- 34: Poröses Material

## Patentansprüche

1. Prüfkörper (1), insbesondere zur Überprüfung der Penetrationseigenschaften eines Sterilisiermittels, bei dem ein zur Aufnahme eines Indikators (26) vorgesehenes Detektorvolumen (24) über einen Gassammelraum (4) mit einer Sterilisationskammer verbindbar ist, **dadurch gekennzeichnet, dass** der Gassammelraum (4) mehrstufig ausgeführt ist, wobei der Querschnitt und das Volumen jeder Stufe (12, 14, 16) zwischen benachbarten Stufen (12, 14, 16) in Richtung zum Detektorvolumen (24) abnehmen.

2. Prüfkörper (1) nach Anspruch 1, bei dem der Querschnitt zwischen benachbarten Stufen (12, 14, 16) in Richtung zum Detektorvolumen (24) mindestens um 50 %, vorzugsweise um mehr als 75 %, abnimmt.

3. Prüfkörper (1) nach Anspruch 1 oder 2, bei dem der Querschnitt der dem Detektorvolumen (24) unmittelbar benachbarten Stufe des Gassammelraums (4) 1 bis 200 mm² beträgt.

4. Prüfkörper (1) nach einem der Ansprüche 1 bis 3, bei dem die dem Detektorvolumen (24) unmittelbar benachbarte Stufe des Gassammelraums (4) eine Kanallänge von zumindest 10 cm, vorzugsweise von etwa 30 bis 100 cm, aufweist.

5. Prüfkörper (1) nach einem der Ansprüche 1 bis 4, bei dem eine Stufe (14) des Gassammelraums (4) innerhalb einer weiteren Stufe (16) des Gassammelraums (4) angeordnet ist.

6. Prüfkörper (1) nach Anspruch 5, bei dem die weitere Stufe (16) des Gassammelraums (4) durch ein die erste Stufe (14) umschließendes Außengehäuses (30) gebildet ist.

7. Prüfkörper (1) nach einem der Ansprüche 1 bis 4, bei dem eine Stufe (14) des Gassammelraums (4) um ein eine weitere Stufe (16) des Gassammelraums (4) bildendes Außengehäuse (30) herumgeführt ist.

8. Prüfkörper (1) nach einem der Ansprüche 1 bis 7, bei dem zumindest eine Stufe (14, 16) des Gassammelraums (4) mit porösem Material (34) gefüllt ist.

9. Prüfkörper (1) nach einem der Ansprüche 1 bis 8, bei dem das Detektorvolumen (24) kleiner als 500 µl gewählt ist.

10. Prüfkörper (1) nach einem der Ansprüche 1 bis 9, in dessen Detektorvolumen (24) als Indikator (26) ein Chemo- oder ein Bio-Indikator angeordnet ist.

## Claims

1. Test device (1), especially to test the penetration characteristics of a sterilization agent with a detector-volume (24) designed to house an indicator (26) connected to a sterilization chamber via a gas-collection-volume (4) **characterized by** the gas-collection-volume being multi-stage-designed so that both cross section and volume of each stage (12, 14, 16) decrease between neighbouring volumes (12, 14, 16) towards the direction of the detector volume.

2. Test device (1) according to claim 1, where the cross section between neighbouring volumes (12, 14, 16) decreases towards the direction of the detector volume (24) by a minimum of 50%, better more than 75%.

3. Test device (1) according to claim 1 or 2, where the cross section of the gas-collection-volume (4) directly adjacent to the detector volume (24) is ca. 1 to 200 mm².

4. Test device (1) according to one of the claims 1 to 3, where the gas-collection-volume (4) directly adjacent to the detector volume (24) has a channel length of minimum 10 cm, better ca. 30 to 100 cm.

5. Test device (1) according to one of the claims 1 to 4, where one stage (14) of the gas-collection-volume (4) is arranged within another stage (16) of the gas-collection-volume (4).

6. Test device (1) according to claim 5, where the next stage (16) of the gas-collection-volume (4) is formed by an outside case (30) enclosing the first stage (14).

7. Test device (1) according to one of the claims 1 to 4, where one stage (14) of the gas-collection-volume (4) is built around an outside case (30) which forms another stage (16) of the gas-collection-volume (4).

8. Test device (1) according to one of the claims 1 to 7, where at least one stage (14, 16) of the gas-collection-volume (4) is filled with porous material (34).

9. Test device (1) according to one of the claims 1 to 8, where the detector volume (24) is selected to be smaller than 500 µl.

10. Test device (1) according to one of the claims 1 to 9, where a chemical or biological indicator is used as an indicator (26).

## Revendications

1. Corps de contrôle (1 ), destiné en particulier au contrôle des caractéristiques de pénétration d'un produit stérilisant, dans lequel un volume détecteur (24) conçu pour recevoir un indicateur (26) peut être relié à une chambre de stérilisation par un collecteur de gaz (4),
**caractérisé par le fait**
**que** le collecteur de gaz (4) comprend plusieurs étapes telles que section et volume de chaque étape (12, 14, 16) entre les étapes voisines (12, 14, 16) vont en décroissant en direction du volume détecteur (24).

2. Corps de contrôle (1) selon spécification 1, dans lequel la section diminue entre les étapes (12, 14, 16) en direction du volume détecteur (24) d'au moins 50%, de préférence plus de 75%.

3. Corps de contrôle (1) selon spécification 1 ou 2, dans lequel la section de l'étape du collecteur de gaz (4) immédiatement voisine du volume détecteur (24) mesure environ de 1 à 200 mm².

4. Corps de contrôle (1) selon l'une des spécifications 1, 2 ou 3, dans lequel l'étape du collecteur de gaz (4) immédiatement voisine du volume détecteur (24) possède une longueur de canal d'au moins 10 cm, de préférence entre 30 et 100 cm.

5. Corps de contrôle (1) selon l'une des spécifications 1, 2, 3 ou 4, dans lequel une étape (14) du collecteur de gaz (4) se situe à l'intérieur d'une étape complémentaire (16) du collecteur de gaz (4).

6. Corps de contrôle (1) selon la spécification 5, dans lequel l'étape complémentaire (16) du collecteur de gaz (4) est formée par un boîtier extérieur (30) englobant la première étape (14).

7. Corps de contrôle (1) selon l'une des spécifications 1 à 4, dans lequel une étape (14) du collecteur de gaz (4) contourne un boîtier extérieur (30) constituant une étape complémentaire (16) du collecteur de gaz.

8. Corps de contrôle (1) selon l'une des spécifications 1 à 7, dans lequel au moins une étape (14, 16) du collecteur de gaz (4) est rempli d'un matériau poreux (34).

9. Corps de contrôle (1) selon l'une des spécifications 1 à 8, dans lequel le volume détecteur (24) choisi est inférieur à 500 µl.

10. Corps de contrôle (1) selon l'une des spécifications 1 à 9, prévoyant comme indicateur (26 ) un indicateur chimique ou biologique.
